Europäisches Patentamt

European Patent Office

Office européen des brevets

(11) Publication number: **0 474 123 A1**

## EUROPEAN PATENT APPLICATION

(21) Application number: **91114569.6**

(22) Date of filing: **29.08.91**

(51) Int. Cl.5: **B32B 5/26**, D04H 13/00, D04H 1/54

(30) Priority: **06.09.90 US 578695**

(43) Date of publication of application:
**11.03.92 Bulletin 92/11**

(84) Designated Contracting States:
**BE DE ES FR GB IT NL SE**

(71) Applicant: **KIMBERLY-CLARK CORPORATION**
**401 North Lake Street**
**Neenah ,Wisconsin 54956-0349(US)**

(72) Inventor: **Mathis, Michael Peter**
**4315 Sandy Plains Road**
**Marietta, GA 30066(US)**

(74) Representative: **Diehl, Hermann Dr. et al**
**Diehl & Glaeser, Hiltl & Partner**
**Flüggenstrasse 13**
**W-8000 München 19(DE)**

(54) Extrusion coated nonwoven laminate, process of making and use of same.

(57) A spunbond-meltblown-fiber composite laminate (10) and a process for making same. A spunbonded mat (12) is ultrasonically bonded to a meltblown web (16). The side (18) of the meltblown web (16) opposite the spunbond mat (12) is extrusion coated with a continuous layer (26) of polymeric material which adheres to the fibers of the meltblown web (16) and forms a fluid impervious backing layer.

FIG. I

The present invention relates to an extrusion coated nonwoven laminate and a process of making and use of same.

The uses to which nonwovens are employed is constantly increasing. As their level of incorporation into products expands, the functions which the nonwovens must perform likewise increases. Oftentimes, no one type of material will suit all the functions necessary to meet the particular requirements of the end product.

One solution to this problem is the use of laminates. This is particularly true with respect to the Health Care industry and, in particular, surgical room supplies including surgical drapes and gowns. Many of the items used in surgery today originated from reusable woven cloth materials which were washed and sterilized after each use. As the nonwoven market has grown, many of these reusable items have been replaced with single use disposable nonwovens. Many of these nonwovens have proven to be great improvements over the use of cloth materials. Perhaps the only advantage of cloth was the fact that it was extremely breathable and therefore relatively comfortable to wear. The primary disadvantage of cloth was that it readily absorbed body fluids including blood. As a result, with the recent and ever growing concern surrounding infectious disease contaminated blood, the barrier and fluid handling characteristics of surgical room materials as well as their physical integrity have become more important issues. It is therefore an object of the present invention to provide a nonwoven-based material which will provide good abrasion resistance, absorbency and fluid barrier properties within the same material while still providing good strength and delamination properties. In order to solve this and related objects, the invention provides an extrusion coated nonwoven laminate according to independent claim 1 and a method of making such a laminate according to independent claim 2, and its use according to independent claim 5. Further advantageous features, aspects and details of the invention are evident from the dependent claims, the description, example and drawings. The claims are intended to be understood as a first non-limiting approach of defining the invention in general terms.

The invention provides a spunbond-meltblown-film composite laminate. More specifically, the present invention provides a prebonded spunbond mat which is ultrasonically bonded to a meltblown web. The side of the meltblown web opposite the spunbond mat is extrusion coated with a continuous layer of polymeric material which adheres to the fibers of the meltblown web and forms a fluid impervious backing layer. The resultant material is suited for a wide variety of uses not the least of which includes surgical and clean room goods such as surgical drapes, gowns and work wear as well as personal care products including diapers, sanitary napkins, training pants, incontinence products and the like.

It is yet another aspect of the present invention to provide a nonwoven material which, because of its materials and methods of construction, will provide these benefits within a single composite structure. These and other aspects of the present invention will become more apparent upon a further review of the following specification, drawings and claims.

The present invention provides a spunbond-meltblown-film composite laminate and a process for making the same. More specifically, the present invention provides a prebonded spunbond mat which is ultrasonically bonded to a meltblown web. The side of the meltblown web opposite the spunbond mat is extrusion coated with a continuous layer of polymeric material which adheres to the fibers of the meltblown web and forms a fluid impervious backing layer. The resultant material is suited for a wide variety of uses not the least of which includes surgical and clean room goods such as surgical drapes, gowns and work wear as well as personal care products including diapers, sanitary napkins, training pants, incontinence products and the like.

The process for forming the extrusion coated nonwoven laminate comprises positioning a mat of continuous and randomly deposited thermoplastic fibers adjacent a web of thermoplastic microfibers. The web is defined as having a top surface and a bottom surface with the mat being positioned adjacent the top surface of the web. Next the web and the mat are ultrasonically bonded together through a plurality of bond sites to create a total bond area less than or equal to 15 percent of the surface area of either the web or the mat. Following the ultrasonic bonding, there is extruded a coating of material onto the bottom surface of the web to create a continuous layer of liquid impervious film thereon. If desired, the mat can be point bonded prior to positioning the mat adjacent the web. The prebonding of the mat is greater than or equal to 20 percent of the total surface area of the mat.

Figure 1 is a cross-sectional side view of a spunbond-meltblown-film composite laminate according to the present invention.

Figure 2 is a schematic diagram of a process for forming the material of a present invention.

Figure 3 is a photomicrograph showing a cross-sectional side view of a spunbond-meltblown-film composite laminate material according to the present invention.

The present invention relates to a composite nonwoven laminate having a layer of spunbond material ultrasonically bonded to a meltblown layer with a fluid impervious layer of film being extrusion coated onto

2

the side of the meltblown layer opposite that of the spunbond layer. For purposes of illustration only the present invention will be described in conjunction with its use in a surgical drape, however, this should not be construed as a limitation as to the use of the present invention. To the contrary, the material of the present invention is adaptable for a wide variety of uses.

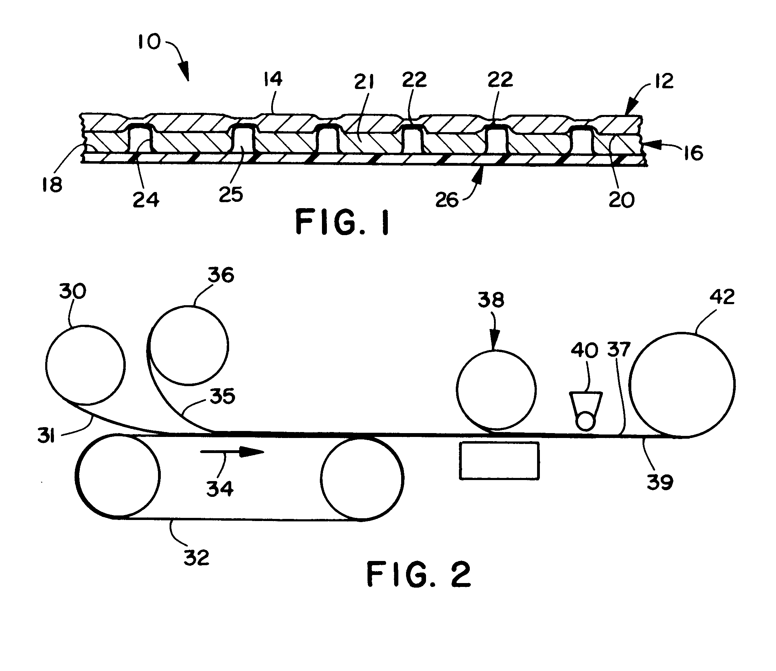

Referring to Figure 1, the top-most layer 12 of the composite laminate 10 according to the present invention is a layer of randomly deposited thermoplastic continuous fibers. Such materials and their generation are well known in the art and are commonly referred to as spunbonded nonwovens. See for example U.S. Patent Number 4,405,297 which discloses the generation of such spunbonded materials. The purpose of the spunbond top layer 12 is to provide an abrasion layer and a good work surface. In surgical procedures this is the layer that will most typically come in contact with the operating room personnel as well as the surgical equipment and supplies. As a result, this surface should be made from fibers which have both good wet and dry strength and resist linting. Spunbond materials are appropriate for such desires. In addition, the spunbond layer must provide a fluid transfer mechanism to allow body and irrigation fluids to travel from the top surface 14 of the spunbond layer 12 down through the material into the meltblown middle layer 16 where they may be absorbed and retained.

Any number of extrudable materials may be used to form the fibers which make up the spunbond layer 12 of the overall composite laminate 10. Thermoplastic materials are particularly well suited for the generation of such fibers. Examples of such thermoplastic resins include, but are not limited to, polyesters, polyolefins such as polyethylene and polypropylene, as well as random copolymers and polymer blends.

Fiber diameters of the individual fibers will range from about 10 to about 25 $\mu$m with a preferred range being from 17 to 20 $\mu$m. When using resins which are typically hydrophobic, it will generally be advisable to include a wetting package either within the fiber or as a surface treatment to increase the hydrophilicity of the spunbond layer, thereby making it more acceptable to receiving and transporting fluids. The basis weight of the material for health care and personal care uses will generally range between about 0.5 and about 1.5 ounces per square yard (osy)*, however, it should be readily apparent that this range can be modified to meet the needs of the particular use to which the material is to be applied.

To increase the integrity of the spunbond layer 12, and to aid in processing and handling, the spunbond layer 12 is usually prebonded using a point bond pattern. To insure proper abrasion resistance, the bond area should typically be at least 20% of the total surface area of the material. Such bonding may be achieved by any number of known methods including, but not limited to, heated patterned bonding rolls and ultrasonic bonding. See for example, U.S. Patent Number 4,374,888. Lastly, it should be appreciated that other additives and treatments may be incorporated into the spunbond layer 12 to enhance its functional characteristics.

The middle layer 16 of the composite laminate 10 is comprised of a plurality of randomly deposited discontinuous microfibers having average fiber diameters ranging from about 1.0 to about 5 $\mu$m with a preferred range of from 1.5 to 3.0 $\mu$m. While these fibers are often regarded as being discontinuous, the ratio of there length to width approaches infinity. Such materials are commonly referred to as meltblown nonwovens, the formation of which is well known in the art. See, for example, U.S. Patent Number 3,676,242 to Prentice issued July 11, 1972 or such as described in an article entitled "Superfine Thermoplastic Fibers," appearing in Industrial Engineering Chemistry, Vol. 48., No. 8, pages 1342 to 1346 which describes work done at the Naval Research Laboratories in Washington, D.C. Also see Naval Research Laboratory Report 11437, dated Apr. 15, 1954. As with the spunbond layer, the polymer or resin used to generate the meltblown fibers may be selected from a wide variety of materials provided that the material so chosen can be ultrasonically bonded to the material used to generate the spunbond fibers. Examples of possible resins include, but are not limited to, nylon, polyester, polyolefins such as polyethylene and polypropylene, random copolymers, elastomers and blends. Again for personal care and health care applications, the basis weight of the meltblown layer 16 will range from about 1 ounce per square yard to about 4 ounces per square yard with the preferred range being from 2.5 to 3.0 ounces per square yard. Basis weights outside this range are also permissible depending upon the particular use for the overall composite laminate.

The meltblown middle layer 16 acts as a fluid absorbent for fluids passed to it from the spunbond top layer 12. As a result, in a preferred embodiment, the meltblown layer 16 will not be prebonded prior to its being ultrasonically bonded to the spunbond layer 12. Furthermore, due to the degree of entanglement of the fine fibers of the meltblown layer, bonding is generally not required. As with the spunbond layer, wetting agents and other substances and treatments may be added to the fibers.

A critical step in the production of the composite laminate of the present invention is the bonding of the

* 1 ouce per square yard = 33.9 gram per square meter spunbond layer 12 to the meltblown layer 16. The two layers of the present invention are point bonded to one another using ultrasonic bonding techniques. Because the meltblown layer is generally more lofty than the spunbond layer, point bonding of the two layers usually involves the use of a patterned bond roller on the bottom side 18 of the meltblown layer to compress together the fibers at the bond points so as to enable them to bond to the fibers of the spunbond layer adjacent the top surface 20 of the meltblown layer 16. Applicant has observed that when using conventional thermo point bonding, the bonds within the meltblown layer have tapered walls which themselves have partial fusing and bonding of the fibers due to the heat transferred to the fibers by the patterned points on the heated roller. As a result, excessive bonding takes place which does nothing to enhance the bonding of the meltblown layer 16 to the spunbond layer 12.

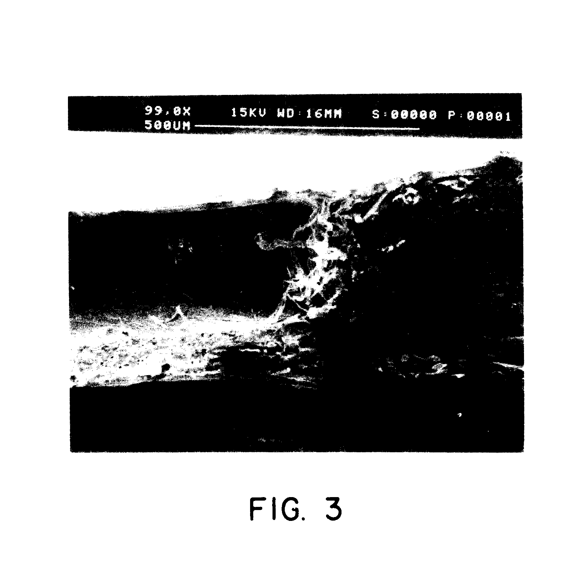

In contrast, by using ultrasonic bonding techniques, a much cleaner bond can be made between the spunbond and meltblown layers. This is because the majority of the bonding only takes place at the actual bond site 22 and not around the periphery of the indentation made by the patterned bond roller. Furthermore, the side walls 24 within the meltblown layer surrounding the bond points 22 are more vertical which, when coupled with the lower degree of bonding in this area, increases the absorptive capacity of the meltblown layer 16 for fluid retention purposes. Ultrasonic bonding also allows the use of incompatible materials with respect to the meltblown and spunbond layers that cannot be joined by thermobonding but can by ultrasonic bonding. Furthermore, the ultrasonic bonding has been found to give higher peel strengths between the meltblown and spunbond layers as compared to similar degrees of bonding using thermobonding techniques. Examples of ultrasonic bonding can be found in U.S. Patent Number 4,605,454. An example of the bonding is shown in the cross-sectional photomicrograph of Figure 3.

Generally speaking the higher the degree of bonding between the spunbond layer 12 and the meltblown layer 16, the lower the absorbency will be due to the compaction and fusing of the meltblown layer to the spunbond layer in the area of the bond sites 22. For general health care and personal care product uses, the degree of bonding will be less than about 15 percent of the total surface area available for bonding between the two layers. Again, though, this range can be expanded to meet particular needs.

Referring again to Figure 1, the bottom side 18 of the meltblown layer 16 has attached thereto a fluid impervious layer 26 which is extrusion coated onto the bottom side 18 of the second layer 16. Extrusion coating the layer 26 is the preferred method of application since, unlike the bonding of a preformed film, extrusion allows the coating to partially coat and therefore bond to and encompass the fibers of the second layer 16 adjacent the bottom side 18. See Figure 3. As a result, the extrusion coated layer 26 may be comprised of a material which would be incompatible with the middle layer 16 if in film form and heat bonded to the meltblown fibers of the middle layer 16.

The material chosen to form the extrusion coated layer 26 should be a material which will readily form a uniform coating across the bottom surface 18 of the meltblown layer 16 so as to form a liquid impervious layer. Suitable materials for extrusion coating include, but are not limited to, polyolefins such as polyethylene and polypropylene, polyethylene blends (EVA and EMA), thermoplastic elastomers, polyurethane and polyester. Application of the extrusion coated layer is by means of casting the extruded film onto the substrate. Typical coating weights will range from about 0.75 to about 2.0 mils**. For example, when low density polyethylene is used as the extrusion material, coating weights between 1.0 and 1.5 mils are suitable to form a liquid impervious layer. Lastly, an additional advantage of extrusion coating over thermo point bonding is the reduced risk of pin holes reaching the layer 26 and thereby permitting the material to become fluid permeable.

As noted earlier, by extrusion coating the layer 26 onto the bottom side 18 of the meltblown layer 16, the extrusion coating is capable of encapsulating the fibers adjacent the surface 18 of the meltblown layer 16. This is shown in Figure 3 which is a photomicrograph of a coating of 1.0 mil of polyethylene extrusion coated onto a 2.65 osy, 2 to 3 micron fiber diameter meltblown layer which in turn has been ultrasonically bonded to a 1.0 osy, 20 micron fiber diameter spunbond layer with the two layers being joined by a 12 percent bond area.

Referring again to Figure 1, once the composite laminate 10 has been formed, it includes a fluid pervious top layer 12 and a fluid impervious bottom layer 26 separated by a fluid absorbent layer 16. The fluid absorbent layer 16 includes a plurality of lofted areas 21 between the bond points 22 separated by a second plurality of voids 25 which are caused by the compaction and bonding of the meltblown layer to the spunbond layer. It is also important to note that the extrusion coated layer 26 coats and surrounds the fibers of the lofted area 21 while also bridging the voids 25 as shown in Figure 3. These voids 25 lie directly below the bond sites 22 and coupled with the lofted areas 21 provide areas for absorbing the fluids which are transmitted thereto via the fluid pervious layer of spunbond material 12. Because the ultrasonic bonding

**1 mil = 0.001 inch; 1 inch = 2.54 cm leaves relatively vertical walls 24 with little or no fusing of the fibers surrounding the bond sites 22, it is possible for the fluid to drain out of the lofted areas 21 and fill these voids 25. It is believed that with the use of a thermobonding process, the degree of fusion would be such that the flow of fluid into these voids would be at least partially impaired due to the fusion of the fibers.

Having thus described the various components of the composite layer according to the present invention and the appropriate means for joining them, a process will now be demonstrated which will permit the formation of such a material.

Referring to Figure 2, there is shown a first supply 30 of a spunbond material 31 being unrolled onto an endless loop conveyor belt 32 rotating in the direction of arrow 34. Next, a second supply 36 of material 35, in this case a roll of meltblown material, is unwound and placed in registry on top the layer of spunbond 31. The layers of spunbond 31 and meltblown 35 are then passed through an ultrasonic bonding means 38 to ultrasonically bond the two layers together with a percent bond area of at least 12 percent. Following the bonding step, an extrusion coater 40 forms a continuous layer of liquid impervious material 37 onto the back side of the meltblown layer 35 thereby forming the composite laminate structure 39 of the present invention. Thereafter, the composite 39 may be wound upon a storage roll 42 so the material may be transported to another location for further processing. Alternatively, such further processing may take place directly in line.

Having thus described both material and the process for making the same, an example will now follow which will demonstrate several of the improved attributes of the present invention.

EXAMPLE 1

The Spunbond/Meltblown/Film Composite Laminate, "SMF", of the present invention was constructed by first producing a 1.0 ounce per square yard spunbond polypropylene mat containing filaments having average diameters of approximately 20 $\mu$m. The spunbond mat was prebonded using heat and pressure with a diamond bond pattern which comprised approximately 26 percent of the mat surface area. Subsequently the web was treated with an anionic surfactant to render the web wettable. Next, a meltblown microfiber web was formed containing filaments having average diameters of 2 to 3 $\mu$m. The web so produced had a basis weight of approximately 2.65 ounces per square yard and was rendered wettable by spraying a nonionic surfactant into the meltblown fiber stream as the web was formed. Rolls of spunbond mat and the meltblown web were then unwound and placed in registry, one on top of the other, before being fed into an ultrasonic bonding unit with the spunbond mat against the ultrasonic horn and the meltblown web against the patterned anvil as energy was applied to effectively "weld" the two materials together. The patterned anvil roll used in the ultrasonic bonding step was a rectangular pin roll with a total bond area of approximately 15 percent. The spunbond/meltblown composite so formed was then wound on rolls, taken to an extrusion coating line, unwound and coated with a 1.0 mil polyethylene film containing 9% ethylene vinyl acetate for softness and 10% pigment concentrate to achieve the desired color.

Referring to Table 1, the characteristics of the spunbond/meltblown/film composite laminate according to the present invention were compared with similar characteristics of three other fabrics labelled "CONTROL@", "HI-LOFT@", and "DRI-SITE@". "CONTROL@" is sold by Kimberly-Clark of Neenah, Wisconsin. "HI-LOFT@" is sold by Convertors, a division of Baxter Health Care of Evansville, Illinois. "DRI-SITE@" is sold by Johnson and Johnson of New Brunswick, New Jersey. The "CONTROL@" is a type of material used as a surgical drape reinforcement fabric and described in Donnelly U.S. Patent No. 3,668,050. As such, it comprises a 1.5 mil polyethylene film adhesively laminated to a 40 mil open celled polyurethane foam. Both "HI-LOFT@" and "DRI-SITE@" are commercially available cellulose-based fenestration reinforcement materials for surgical drapes. Absorbent efficiency, strength, and abrasion resistance are important attributes for such materials when used as a fenestration reinforcement for surgical drapes. As a result, these attributes were measured for all four materials. The methods for evaluating these attributes are set forth below and the results are presented in Table I.

Absorbent Efficiency

This test was preformed according to Federal Government Specification UU-T-595b. In the test, a 4"x 4"\*\*\*sample was cut, weighed, saturated with water for three minutes by soaking, removed from the water and hung by one corner for 30 seconds to allow excess water to drain off and then reweighed. The dry weight was subtracted from the wet weight and the result was divided by the dry weight with the quotient

\*\*\*1 inch = 2.54 cm expressing the efficiency in terms of grams of water per gram of substrate. Thus, a higher number meant a higher absorbent efficiency.

CD Trapezoidal Tear

The cross direction (CD) trapezoidal tear strength of each of the materials was measured using Federal Standard 191, Method 5136. The results in Table I are reported in pounds.

Grab Tensile

The grab tensile in both the machine direction (MD) and cross direction (CD) was calculated for each material using Federal Standard 191, Method 5102. The results in Table I are reported in pounds + +.

Abrasion Resistance

Abrasion resistance measurements were made using a Taber Standard Abrader (Model 140PT) with a rubber calibrate No. S-32 wheel on the right abrading head and a 125 gram counterweight (total load of 125 grams). The results were obtained and reported in abrasion cycles to failure where failure was deemed to occur at that point where a noticeable portion of the surface subjected to abrasion in the test exhibited a fuzzy, pile-like appearance.

TABLE I

|  | "SMF" | CONTROL@ | HI-LOFT@ | DRI-SITE@ |
|---|---|---|---|---|
| Absorbent Efficiency (gms. water/gm. substrate) | 5.10 | 8.65 | 2.52 | 4.53 |
| CD Trap Tear (lbs) | 5.2 | 4.4 | 2.4 | 0.9 |
| MD Grab Tensile (lbs) | 32.8 | 9.1 | 10.5 | 10.6 |
| CD Grab Tensile (lbs) | 25.9 | 9.1 | 7.2 | 11.6 |
| Taber Abrasion (cycles) | 100 + | 28 | 100 + | 22 |

Reviewing the results of the testing in Table I, comparable absorbent efficiency of the "SMF" of the present invention to the other commercially available fenestration reinforcement materials was indicated making the "SMF" suitable for use as a surgical drape material or a surgical drape fenestration reinforcement material.

Strength characteristics, which were reported in terms of both trapezoidal tear and grab tensile, were significantly better than any of the commercially available materials tested and were a direct result of the ultrasonic bonding step. This bond strength is of extreme importance in surgical end uses wherein towel clips are often used to secure surgical tubing to the drape around the area of the incision. Failure of the material to securely anchor the tubing in place as a result of the towel clip tearing out of the fenestration reinforcement material could result in possible injury to the patient.

Abrasion resistance of the "SMF" material was comparable to the best of the currently available commercial fenestration reinforcement materials, again making the material well suited for surgical applications.

**Claims**

**1.** An extrusion coated nonwoven laminate (10) comprising:

a mat (12) of continuous and randomly deposited thermoplastic fibers,

a web (16) of thermoplastic meltblown microfibers having a top surface (20) and a bottom surface (18), said mat being ultrasonically bonded to said top surface of said web with a percent bond area (22) to total surface area less than or equal to fifteen percent, and

+ +1  pound   =  0.453  kg

a continuous film (26) of polymeric material extrusion coated onto said bottom surface (18) of said web.

2. A process for forming an extrusion coated nonwoven laminate especially according to claim 1 comprising:

   (a) positioning a mat of continuous and randomly deposited thermoplastic fibers adjacent a web of thermoplastic microfibers, said web having a top surface and a bottom surface with said mat being positioned adjacent said top surface of said web;
   (b) ultrasonically bonding said mat to said web through a plurality of bond sites to create a total bond area less than or equal to 15% of the surface area of either of said web or said mat, and
   (c) extruding a coating of material onto said bottom surface of said web to create a continuous layer of liquid impervious film thereon.

3. The process of claim 2 which further the steps of point bonding said mat prior to positioning said mat adjacent said web.

4. The process of claim 3 wherein said point bonding creates a total bond area relative to the total surface area of said mat which is greater than or equal to 20% of the total surface area of said mat.

5. Use of an extrusion coated nonwoven laminate according to claim 1 or produced according to one of claims 2 to 4 as or in surgical, clean room and personal care products.

6. Use of a laminate according to claim 5, as or in surgical drapes, gowns and work wear, as well as diapers, sanitary napkins, training pants and incontinence products.

FIG. 1

FIG. 2

EP 0 474 123 A1

FIG. 3

# European Patent Office

## EUROPEAN SEARCH REPORT

Application Number

### EP 91 11 4569

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 245 933 (KIMBERLY-CLARK LIMITED)November 1987<br>– – – | 1,2,5,6 | B 32 B 5/26<br>D 04 H 13/00<br>D 04 H 1/54 |
| A | EP-A-0 245 933 ( )<br>* page 2, line 20 - page 2, line 22; claims 3,5 * * * * page 3, line 49 - page 3, line 54 * * * abstract * *<br>– – – | 3,4 | |
| D,Y | US-A-3 672 242 (ESSO RESEARCH AND ENGINEERING COMPANY) 11 July 1972<br>* column 1, line 56 - column 1, line 59; claims 1,3; example 1 * *<br>– – – | 1,2,5,6 | |
| Y | EP-A-0 325 543 (JAMES RIVER CORPORATION OF VIRGINIA) 26 July 1989<br>– – – | 1 | |
| A | EP-A-0 325 543 ( )<br>* abstract; claims 1,4-6,11-14 * * * column 1, line 5 - column 2, line 10 * * * column 3, line 26 - column 3, line 56 * *<br>– – – | 2,3,5,6 | |
| Y | US-A-4 591 526 (GRANT ET AL.)<br>* column 1, line 64 - column 3, line 3; claims 1,2; example 1 * *<br>– – – | 1 | **TECHNICAL FIELDS SEARCHED (Int. Cl.5)**<br><br>D 04 H<br>B 32 B |
| A | EP-A-0 212 284 (KIMBERLY-CLARK CORPORA-TION)March 1987<br>* page 2, line 16 - page 3, line 32 * * * claims 1,2,5,6 * *<br>– – – | 1,5,6 | |
| A | US-A-4 863 785 (BERMAN ET AL.)<br>* column 1, line 51 - column 2, line 56; claims 1-10; figure 1 * * * column 4, line 13 - column 4, line 52 * *<br>– – – – – | 1,3-6 | |

The present search report has been drawn up for all claims

| Place of search | Date of completion of search | Examiner |
|---|---|---|
| The Hague | 04 November 91 | DERZ T. |